# EUROPEAN PATENT APPLICATION

(11) **EP 1 767 213 A2**
(43) Date of publication of application: **28.03.2007**
(21) Application number: 06118836.3
(22) Date of filing: 22.10.2002
(51) Int. Cl.: A61K 38/29, A61P 43/00

(54) **Method for improving stability of a bone-connecting implant**

(30) Priority: 05.11.2001 US 337958
(62) Divisional of application: 02802774.6
(71) Applicant: Eli Lilly & Company, Indianapolis, IN 46285 (US)
(72) Inventor: Mitlak, Bruce Howard, Indianapolis, IN IN 46240 (US)
(74) Representative: Burnside, Ivan John

(57) **Abstract**

The present invention provides a method for improving the stability of a bone-connecting implant in a human by administering PTH. A kit comprising an informational label and a composition of PTH and a buffer in one or more containers is also provided.

## Description

This invention is in the field of human medicine. In particular, this invention is in the field of pharmaceutical treatments to improve the stability of bone-connecting implants in humans.

Successful implantation and function of bone-connecting implants in humans depends on bonding the adjacent bone to the implant. Such bonding requires bone repair by formation of new matrix components at the interface between the implant and the bone, proximate to the implant.

An estimated ten percent of bone and joint prosthetic devices that are placed in people fail to function due to non-binding of the bone to the implant. The resulting disability often requires reoperation and reimplantation of the device.

A variety of bone cements and porous-coated implant surfaces have been proposed to improve the stability of bone-connecting implants. In addition, pharmaceutical agents such as growth hormone and angiotensin peptides have been proposed to be admixed with bone cements or to be applied directly to implants to improve growth of the bone matrix [Downes, S., WIPO publication WO89/03695, 5 May 1989; and Rodgers, K. et al., U.S. Patent No. 6,258,778 B1 issued July 10, 2001]. Furthermore, as reported by Hilding, M. et al. in Acta Orthopaedica Scandinavica 71:553-557 (2000), the bisphosphonate Clodronate administered orally to patients undergoing total knee replacement appeared to reduce prosthetic migration.

Parathyroid hormone (PTH) is a secreted, 84 amino acid protein of the mammalian parathyroid gland that controls serum calcium levels through its action on various tissues, including bone. The N-terminal 34 amino acids of human PTH (PTH(1-34)) appear to retain the same biological activity as the full length hormone. Administration of PTH(1-34) to humans suffering from osteoporosis has been shown to increase spinal bone mineral content and reduce fractures.

Recently, it has been shown that administration of PTH(1-34) to rats at 15-240 pg/kg/day for six weeks enhances new bone formation in a titanium bone chamber model (Skripitz, R. et al., J. Bone and Joint Surgery 82-B(1), pp. 138-141, 2000) and that administration of PTH(1-34) to rats at 60 pg/kg/day for four weeks enhances fixation of a stainless steel screw (Skripitz, R. et al., J. Bone and Joint Surgery 83(3), pp. 437-440, 2001).

Despite the developments noted above, there remains a need in the art for improved methods of enhancing stability of a bone-connecting implant that are applicable to humans.

Accordingly, one aspect of the present invention is a method of improving stability of a bone-connecting implant in a human comprising, administering to the human about 5 µg to about 100 µg of PTH per day on two or more days during a period from about 6 months before to about 12 months after insertion of the implant. Preferably, the PTH is PTH(1-34) and the dose of PTH(1-34) administered in the treatment regimen is about 5 µg to about 40 µg per day.

Another aspect of the present invention is the use of PTH in the manufacture of a medicament for improving stability of a bone-connecting implant in a human.

Another aspect of the present invention is a kit comprising one or more containers comprising a pharmaceutically acceptable composition of PTH and a buffer to maintain a pH from about 2 to about 8, and a label comprising information indicating the composition may be administered to a human to improve stability of a bone-connecting implant.

For all aspects of the present invention the most preferred PTH is PTH(1-34).

The method of the present invention is useful to strengthen a connection between a bone and an artificial implant device and reduces the need for reoperation or reimplantation of the device.

The term "PTH" means the full length, 84 amino acid form of human parathyroid hormone, designated PTH(1-84), and biologically active fragments thereof, such as PTH(1-28), PTH(1-31), PTH(1-34), PTH(1-37), PTH(1-38) and PTH(1-41). The amino acid sequence of PTH(1-84) is reported by Kimura, T. et al. in Biochem. Biophys. Res. Commun. 114(2):493-499 (1983). Preferably, the PTH is PTH(1-34) or PTH(1-84) and more preferably the PTH is PTH(1-34). PTH may be prepared recombinantly or by peptide synthesis. PTH(1-84) may also be obtained from human fluid.

The term "bone-connecting implant" refers to a device inserted into a person in need thereof and positioned in such a manner that growth of bone matrix occurs between one or more of the person's bones and the device. Examples of bone-connecting implants include a replacement implant for a hip, knee, shoulder or elbow, a metallic spinal vertebrae bridge, and a dental implant. An artificial implant may be constructed from a variety of materials, including titanium, ceramic, steel and synthetic polymers. The exterior of an artificial implant may be porous or non-porous.

The term "improving stability" means reducing the physical displacement, migration or shifting of an implanted device from an adjacent bone surface. This is achieved with use of the present invention by increasing the formation of new bone at the interface between a bone-connecting implant and a bone surface proximate to the implant.

Improved stability of a bone-connecting implant can be most readily demonstrated by measuring post-operative migration of the implant using radiosterometric analysis (RSA). For example, in Hilding, M. et al., Acta Orthopaedica Scandinavica 71:553-557 (2000), several 0.8 mm tantalum markers were positioned in both the proximal tibia and the plastic insert during total knee replacement surgeries. The positions of the markers were followed by radiographs taken one day, six weeks, six months and one year following the operation as a means to monitor postoperative implant shifting. Other techniques for measuring or evaluating the stability of bone-connecting implants are known to those skilled in the art and may be applied to the present invention.

The term "administering" means introducing into a person a designated quantity of PTH. The PTH may be administered by parenteral or non-parenteral routes. Parenteral delivery, which by strict definition means administration of a drug other than through the intestines, includes subcutaneous injection, intravenous injection or infusion, intramuscular injection, pulmonary inhalation, buccal delivery and transdermal delivery. Preferably, for the present invention the PTH is administered subcutaneously. Non-parenteral routes of administration include oral delivery.

The term "strengthening" or "strengthened connection" refers to improved bone density and bone architecture such that the bone is more resistant to mechanical stress.

For the present invention, the term "per day" means each distinct but not necessarily consecutive 24-hour time period in which PTH is administered. Terms such as "every day" or "on consecutive days" are used to indicate a treatment regimen in which the PTH is administered during consecutive 24-hour time periods.

The term "pharmaceutically acceptable composition" means a formulation comprising PTH that is suitable for pharmaceutical use in humans. A composition may be in any form, such as a solution, suspension, freeze-dried pellet, oil, cream, tablet, capsule or ointment. Preferably, the composition is in a sterile, ready to use solution form. A composition of PTH may be available for administration in any of a variety of containers, including a vial, bottle, jar, cartridge or pen injector device.

The term "pharmaceutically acceptable carrier" means a solid or liquid chemical that is essentially inactive pharmacologically but is compatible with and suitable for pharmaceutical use in humans. Examples of pharmaceutically acceptable carriers include water, mannitol and lactose. A preferred carrier is water.

The term "label" refers to written or printed matter accompanying an article of manufacture or kit that provides information about a pharmaceutical product and its use. A label may consist of one or more sheets, documents or forms. For pharmaceutical products provided as a kit, a label may be affixed to a container comprising an active pharmaceutical ingredient. A label may also be co-located with one or more suitable containers comprising an active pharmaceutical ingredient in a small box located within a kit. A label may also be loosely positioned within a kit. Other locations or multiple locations of one or more identical or different labels are also possible. The objective of a label in a pharmaceutical kit is to provide information useful to a patient, health care provider, and/or others involved in using an active pharmaceutical ingredient to treat the patient. The term "label" as applied to the present invention would provide information to health care providers and/or patients as to the therapeutic use of PTH to improve and/or strengthen bone implants following implant surgery.

The present invention is applicable to human subjects having a need for a bone-connecting implant. Certain groups of subjects may find particular benefit from this invention. Bone-connecting implants are often utilized in elderly patients who are in need of the implant due to bone-related diseases such as osteoporosis. The present invention is also applicable to patients undergoing bone-connecting implant procedures due to degenerative diseases such as osteoarthritis, due to inflammatory diseases such as rheumatoid arthritis, and due to traumatic injuries such as those resulting from car accidents or sports-related activities. Thus, although most of the human subjects benefiting from the present invention will be elderly, it is also useful to patients under age 60, younger adults such as those under age 40, and even children.

Certain patient populations are at an increased risk of implant failure due to non-binding of the bone to the implant and will therefore receive particular benefit from the present invention. These groups include, *inter alia,* patients who have diabetes or vascular disease, those who are on long-term glucocorticoid therapy, and those who smoke (see Cook, S. D. et al., Clinical Orthopaedics and Related Research 337, pp. 198-207, 1997).

One aspect of the present invention is a method of improving stability of a bone-connecting implant in a human, comprising administering to the human about 5 µg to about 400 µg of PTH per day on two or more days during a period from about 6 months before to about 12 months after insertion of the implant. Preferably, about 5 µg to about 100 µg of PTH is administered per day during a period from about 6 months before to about 12 months after insertion of the implant. Preferably, PTH is administered on two or more days during a period from about 6 months before to about 6 months after insertion of the implant. Preferably, PTH is administered once every day for about seven or more consecutive days. More preferably, PTH is administered once every day for about 30 or more consecutive days. Most preferably, PTH is administered once every day for about 60 or more consecutive days.

Preferably, administration of PTH according to the present invention begins during a period from about 1 month before insertion of the bone-connecting implant to the day of insertion of the implant. More preferably, administration of PTH begins during a period from about 3 months before insertion of the implant to the day of insertion of the implant. Most preferably, administration of PTH begins during a period from about 6 months before insertion of the implant to the day of insertion of the implant.

Under some circumstances, such as insertion of a bone-connecting implant due to acute trauma, treatment with PTH prior to the day of insertion of the implant may not be possible. In cases in which treatment with PTH does not begin prior to the day of implantation, PTH treatment according to the present invention preferably begins on the day the bone-connecting implant is inserted into the body or within the subsequent 2 months. More preferably, PTH treatment begins on the day of implantation or within the subsequent 10 days. Most preferably, PTH treatment begins on the day of implantation or within the subsequent 2 days.

Treatment with PTH may continue after stabilization of a bone-connecting implant within a patient is essentially complete, and even after about 12 months following implant surgery, for example, to maintain bone mass and strength and/or to further enhance the mass or density of a patient's skeletal bones in general.

The present invention includes treatment regimens in which patients being administered PTH as described herein are administered concurrently one or more other drugs and/or vitamin supplements useful for treating or preventing osteoporosis, for decreasing the incidence of bone fractures, for increasing bone mineral density, or for addressing any other of their medical needs. Such concurrently administered medications include, *inter alia,* raloxifene hydrochloride, calcium, vitamin D, bisphosphonates, and estrogen.

Preferably, the PTH used in a treatment regimen according to the present invention is PTH(1-34) and it is administered at a dose of about 5 µg to about 100 µg per day. More preferably, the dose of PTH(1-34) administered is about 5 µg to about 40 µg per day. More preferably, the dose of PTH(1-34) administered is about 20 µg to about 40 µg per day. Most preferably, the dose of PTH(1-34) administered is about 20 µg per day.

It is well known that for molecules such as PTH, non-parenteral administration such as oral delivery usually results in a much lower level of absorption and bioavailability than delivery by parenteral routes such as subcutaneous injection. Therefore, when PTH is administered non-parenterally according to the present invention, about 40 µg to about 400 µg of PTH is provided to a patient per day. Preferably, when administered non-parenterally, about 40 µg to about 200 µg per day of PTH is provided to a patient. More preferably, when administered non-parenterally, about 40 µg to about 100 µg per day of PTH is provided to a patient.

A dose of PTH administered to a patient in need thereof may also be calculated based on the body weight of the patient. For the present invention, when PTH is delivered parenterally, less than about 10 pg/kg per day is administered to the patient and, preferably, less than about 5 pg/kg per day is administered. More preferably, less than about 2 pg/kg of PTH is administered parenterally per day and most preferably, less than about 1 pg/kg of PTH is administered parenterally per day.

One skilled in the art will recognize that the present invention may encompass numerous and varied treatment regimens. This variety includes the amount of PTH administered, the route and site of administration, the specific PTH peptide utilized, the form of the PTH composition, the number of days the PTH composition is administered, the intervals, if any, between each day of PTH administration, the type and structure of the bone-connecting implant utilized and the type of patient being treated. Each treatment option may operate independently and may be adjusted as needed to improve the stability of the bone-connecting implant.

For example, a male or female suffering from osteoarthritis and in need of a hip replacement procedure is injected subcutaneously in the thigh or abdomen with an aqueous solution comprising 20 µg of PTH(1-34) every day for about six months prior to the implant surgery, then, beginning two days after the surgery, is injected subcutaneously in the thigh or abdomen with 20 µg of PTH(1-34) every day for about six months.

In all aspects of the present invention, the PTH is preferably PTH(1-34), the amount of PTH administered per day is preferably 20 µg, and the PTH is preferably administered parenterally. More preferably, the PTH is administered subcutaneously.

Another aspect of the present invention is the use of PTH in the manufacture of a medicament for improving stability of a bone-connecting implant in a human. The PTH is preferably PTH(1-34). The medicament may be in any form, such as a solution, suspension, pellet, oil, cream, tablet, capsule or ointment. Preferably, the medicament is a sterile, ready to use solution. One skilled in the art will recognize that a variety of medicament forms and procedures for their preparation are available. A suitable medicament would comprise PTH and a buffer to maintain a pH from about 2 to about 8; preferably a pH from about 3 to about 7; more preferably a pH from about 4 to about 5. Suitable buffers for a solution formulation include acetate, tartrate or citrate. Suitable buffers for other than a solution formulation include acetate, tartrate, citrate or phosphate. An example of a medicament is an aqueous solution comprising, per milliliter, about 250 µg of PTH(1-34), about 0.41 mg acetic acid, about 0.1 mg sodium acetate, about 46.4 mg mannitol and about 3.0 mg m-cresol.

The present invention also relates to a kit having one or more containers wherein at least one of the containers comprises a pharmaceutically acceptable composition comprising PTH and a label having recorded thereon information indicating the composition may be administered to a human to improve stability of and/or strengthen a bone-connecting implant.

In a kit according to the present invention the PTH is preferably PTH(1-34). The PTH composition may be in any pharmaceutically acceptable form, such as a sterile, ready to use solution, freeze-dried pellet, suspension, oil, cream, tablet, capsule or ointment. The composition is preferably in solution form. In a solution composition, the PTH is preferably at a concentration of about 100 ug/mL to about 500 ug/mL, more preferably at a concentration of about 200 ug/mL to about 300 ug/mL, and most preferably at a concentration of about 250 ug/mL. The PTH composition may also comprise a pharmaceutically acceptable carrier. Examples of pharmaceutically acceptable carriers include water, mannitol, lactose and mixtures thereof. A preferred carrier is water.

Other pharmaceutically acceptable excipients well known to those skilled in the art may also be incorporated into a composition according to this aspect of the invention. Such excipients include isotonicity agents such as glycerin and sodium chloride; preservatives such as benzyl alcohol, m-cresol, phenol and methylparaben; bulking or stabilizing agents such as lactose and mannitol; buffers such as sodium phosphate, sodium citrate and glycine; and pH modifying agents such as hydrochloric acid, acetic acid and sodium hydroxide.

An example of a composition in a kit according to this aspect of the invention is an aqueous solution comprising, per milliliter, about 250 µg of PTH(1-34), about 0.41 mg acetic acid, about 0.1 mg sodium acetate, about 46.4 mg mannitol and about 3.0 mg m-cresol.

A kit according to this aspect of the invention comprises one or more sealed containers comprising a composition of PTH. Numerous types of containers suitable for holding the PTH composition are well known in the art and include a vial, a cartridge, a syringe, and a bottle.

An essential component of a kit according to this aspect of the invention is a label comprising information indicating the PTH composition may be administered to a human to improve the stability of, or alternatively, to strengthen a bone-connecting implant. The label may also comprise information indicating the quantity of PTH that may be suitably administered to a patient, as well as one or more appropriate administration regimens, to improve the stability of a bone-connecting implant. The label may also comprise information instructing a patient, health care provider, or others involved in using the active pharmaceutical ingredient how the composition is to be prepared for administration to the patient.

A kit according to this aspect of the invention may also comprise a container comprising a pharmaceutically acceptable reconstituting solution. Part or all of the reconstituting solution is combined with a solution, suspension or solid form of the PTH which is located in a separate container within the kit. An example of this type of kit is one comprising a first vial of lyophilized PTH(1-34) and a second vial comprising a reconstituting solution such as bacteriostatic water for injection. In this example, a label within the kit provides information indicating how the lyophilized PTH(1-34) is to be combined with part or all of the bacteriostatic water for injection prior to administration of the reconstituted PTH(1-34) composition to a patient. A kit may also comprise one or more syringes, either empty or pre-filled with a diluent, for use in combining and/or reconstituting the PTH with the diluent. Many other configurations and optional components of a kit according to this aspect of the invention are operable and well known to those skilled in the art.

### Example 1

A 74-year old woman weighing 75 kg presents with a high level of pain on the left side of her hip. The patient history reveals a prior diagnosis of osteoporosis. A thorough examination including X-rays leads to a recommendation of hip replacement surgery due to degenerative arthritis.

Table 1 summarizes a therapeutic regimen to improve the stability of a bone-connecting implant to be placed in the patient described above. Treatment days prior to insertion of the implant are designated by negative numbers; treatment on the day of implantation is designated as day 0; and treatment days subsequent to the day of implantation are designated by positive numbers.

**Table 1**

| **Treatment Overview for Example 1** | |
|---|---|
| Patient profile | Female, Age 74 |
| Patient history | Osteoporosis |
| Implant device | Artificial hip |
| PTH to be utilized | PTH(1-34) |
| PTH treatment regimen | Days -50 to -1 (40 µg every day) |
| | Day 0 (none) |
| | Days 1 to 67 (20 µg every day) |
| PTH administration | Subcutaneous, in the abdomen or thigh |
| PTH medicament | Aqueous solution comprising 250 µg/mL of PTH(1-34), 0.41 mg/mL acetic acid, 0.1 mg/mL sodium acetate, pH 3-7 |

The displacement or migration of the implanted artificial hip is measured over a 6-month period using radiosterometric analysis (RSA) as described in Hilding et *al., supra.* Treatment of the patient with PTH as described in Table 1 is expected to improve the stability of the bone-connecting implant as measured by the RSA technique and reduce the likelihood of reoperation.

### Example 2

A 64-year old man weighing 60 kg presents with a history of osteoarthritis and pain on the left side of his hip. A thorough examination including X-rays and MRI scans leads to a recommendation of hip replacement surgery. Table 2 summarizes a therapeutic regimen to improve the stability of the bone-connecting implant to be placed in the patient described above. Treatment days prior to insertion of the implant are designated by negative numbers; treatment on the day of implantation is designated as day 0; and treatment days subsequent to the day of implantation are designated by positive numbers.

**Table 2**

| **Treatment Overview for Example 2** | |
|---|---|
| Patient profile | Male, Age 64 |
| Patient history | Osteoarthritis |
| Implant device | Artificial hip |
| PTH to be utilized | PTH(1-34) |
| PTH treatment regimen | Days -40 to -1 (20 µg every other day) |
| | Day 0 (20 µg) |
| | Days 1 to 140 (20 µg every day) |
| PTH administration | Subcutaneous, in the thigh or abdomen |
| PTH medicament | Aqueous solution comprising 250 µg/mL of PTH(1-34), 0.41 mg/mL acetic acid, 0.1 mg/mL sodium acetate, 46.4 mg/mL mannitol and 3.0 mg/mL m-cresol |

The displacement or migration of the implanted artificial hip is measured over a 9-month period using radiosterometric analysis (RSA) as described in Hilding et *al., supra.* Treatment of the patient with PTH as described in Table 2 is expected to improve the stability of the bone-connecting implant as measured by the RSA technique and reduce the likelihood of reoperation.

### Example 3

A 14-year old male weighing 40 kg presents with multiple fractures of the left tibia resulting from a car accident. The patient history reveals no prior evidence of bone or cartilage disease. A thorough examination including X-rays leads to a recommendation of immediate tibia replacement surgery.

Table 3 summarizes a therapeutic regimen to improve the stability of the bone-connecting implant to be placed in the patient described above. Treatment days prior to insertion of the implant are designated by negative numbers; treatment on the day of implantation is designated as day 0; and treatment days subsequent to the day of implantation are designated by positive numbers.

**Table 3**

| **Treatment Overview for Example 3** | |
|---|---|
| Patient profile | Male, Age 14 |
| Patient history | Traumatic injuries, otherwise healthy |
| Implant device | Tibia replacement |
| PTH to be utilized | PTH(1-34) |
| PTH treatment regimen | Day 0 (40 µg) |
| | Day 1 (40 µg) |
| | Days 2 to 90 (20 µg every day) |
| PTH administration | Subcutaneous, in the thigh or abdomen |
| PTH medicament | Aqueous solution comprising 250 µg/mL of PTH(1-34), 0.41 mg/mL acetic acid, 0.1 mg/mL sodium acetate, 46.4 mg/mL mannitol and 3.0 mg/mL m-cresol |

The displacement or migration of the implanted tibia is measured over a 4-month period using radiosterometric analysis (RSA) as described in Hilding *et al., supra.* Treatment of the patient with PTH as described in Table 3 is expected to improve the stability of the bone-connecting implant as measured by the RSA technique and reduce the likelihood of reoperation.

### Example 4

A 64-year old woman weighing 55 kg and with a history of osteoporosis presents with moderate pain and swelling on the left side of her leg. The patient mentions a recent severe fall on an icy sidewalk. A thorough examination including X-rays and MRI scans leads to a recommendation of knee replacement surgery within a few days.

Table 4 summarizes a therapeutic regimen to improve the stability of the bone-connecting implant to be placed in the patient described above. Treatment days prior to insertion of the implant are designated by negative numbers; treatment on the day of implantation is designated as day 0; and treatment days subsequent to the day of implantation are designated by positive numbers.

**Table 4**

| **Treatment Overview for Example 4** | |
|---|---|
| Patient profile | Female, Age 64 |
| Patient history | Osteoporosis, traumatic injury |
| Implant device | Knee replacement |
| PTH to be utilized | PTH(1-34) |
| PTH treatment regimen | Days -3 to -1 (20 µg every day) |
| | Day 0 (none) |
| | Days 1 to 20 (100 µg every day) |
| | Days 21 to 200 (100 µg every other day) |
| PTH administration | Subcutaneous (Days -3 to -1) Oral (Days 1 to 200) |
| PTH medicament | Subcutaneous: Same as in Table 3 Oral: Gelatin capsule comprising 100 µg of PTH(1-34) |

The displacement or migration of the implanted artificial knee is measured over a 12-month period using radiosterometric analysis (RSA) as described in Hilding et *al., supra.* Treatment of the patient with PTH as described in Table 4 is expected to improve the stability of the bone-connecting implant as measured by the RSA technique and reduce the likelihood of a reoperation.

### Example 5

A 58-year old woman weighing 80 kg presents with severe pain in her left hip resulting from a fall. The patient has been a pack-per-day cigarette smoker for more than 40 years and has type 2 diabetes currently being treated with insulin. A thorough examination including X-rays leads to a recommendation of hip replacement surgery.

Table 5 summarizes a therapeutic regimen to improve the stability of the bone-connecting implant to be placed in the patient described above. Treatment days prior to insertion of the implant are designated by negative numbers; treatment on the day of implantation is designated as day 0; and treatment days subsequent to the day of implantation are designated by positive numbers.

**Table 5**

| **Treatment Overview for Example 5** | |
|---|---|
| Patient profile | Female, Age 58 |
| Patient history | Type 2 diabetes, heavy smoker |
| Implant device | Artificial hip |
| PTH to be utilized | PTH(1-34) |
| PTH treatment regimen | Days -7 to -1 (20 µg every day) |
| | Day 0 (none) |
| | Days 1 to 40 (100 µg every day) |
| | Days 41 to 180 (100 µg every other day) |
| PTH administration | Subcutaneous, in the thigh or abdomen |
| PTH medicament | Aqueous solution comprising 250 µg/mL of PTH(1-34), 0.41 mg/mL acetic acid, 0.1 mg/mL sodium acetate, 46.4 mg/mL mannitol and 3.0 mg/mL m-cresol |

The displacement or migration of the implanted artificial hip is measured over a 6-month period using radiosterometric analysis (RSA) as described in Hilding et *al., supra.* Treatment of the patient with PTH as described in Table 5 is expected to improve the stability of the bone-connecting implant as measured by the RSA technique and reduce the likelihood of reoperation.

## Claims

1. The use of PTH in the manufacture of a medicament for improving stability of a bone-connecting implant in a human.

2. The use of Claim 1, wherein the PTH is PTH(1-34).

3. A method of improving stability of a bone-connecting implant in a human comprising, administering to said human about 5 µg to about 100 µg of PTH per day on two or more days during a period from about 6 months before to about 12 months after insertion of said implant into said human.

4. The method of Claim 3, wherein administration of the PTH begins during a period from about 3 months before insertion of the implant to the day of insertion of the implant.

5. The method of Claim 3, wherein the PTH is PTH(1-34).

6. The method of Claim 5, wherein about 5 µg to about 40 µg of PTH is administered per day.

7. The method of Claim 6, wherein about 20 µg to about 40 µg of PTH is administered per day.

8. The method of Claim 7, wherein about 20 µg of PTH is administered per day.

9. The method of Claim 3, wherein the PTH is administered parenterally.

10. The method of Claim 9, wherein the PTH is administered subcutaneously.

11. A kit comprising:
(a) one or more containers comprising a pharmaceutically acceptable composition of PTH and a buffer to maintain a pH from about 2 to about 8; and
(b) a label comprising information indicating said composition may be administered to a human to improve stability of a bone-connecting implant.

12. The kit of Claim 11, wherein the PTH is PTH(1-34).

13. The kit of Claim 11, wherein the composition further comprises a pharmaceutically acceptable carrier.

14. The kit of Claim 13, wherein the composition is in solution form and the carrier is water.

15. The kit of Claim 14, wherein each milliliter of the aqueous solution comprises about 250 µg of PTH(1-34), about 0.41 mg acetic acid, about 0.1 mg sodium acetate, about 46.4 mg mannitol, and about 3.0 mg m-cresol.
